(19) Europäisches Patentamt / European Patent Office / Office européen des brevets

(11) **EP 1 164 186 B1**

(12) **EUROPÄISCHE PATENTSCHRIFT**

(45) Veröffentlichungstag und Bekanntmachung des Hinweises auf die Patenterteilung:
**23.07.2008 Patentblatt 2008/30**

(51) Int Cl.:
*C12M 1/34* (2006.01)   *G01N 27/414* (2006.01)

(21) Anmeldenummer: **01110520.2**

(22) Anmeldetag: **28.04.2001**

(54) **Verfahren zur Untersuchung von membranumschlossenen Biokompartimenten**

Method for investigating membrane enclosed biocompartments

Méthode pour l'examen de compartiments biologiques entourés par des membranes

(84) Benannte Vertragsstaaten:
**DE FR GB IT NL**

(30) Priorität: **09.06.2000 DE 10028692**

(43) Veröffentlichungstag der Anmeldung:
**19.12.2001 Patentblatt 2001/51**

(73) Patentinhaber: **Micronas GmbH**
**79108 Freiburg i. Br. (DE)**

(72) Erfinder: **Lehmann, Mirko, Dipl.-Phys.**
**79102 Freiburg (DE)**

(74) Vertreter: **Huwer, Andreas**
**Grünwälderstrasse 10-14**
**79098 Freiburg i. Br. (DE)**

(56) Entgegenhaltungen:
**WO-A-90/04645**

• **BAUMANN W H ET AL: "Microelectronic sensor system for microphysiological application on living cells" SENSORS AND ACTUATORS B, ELSEVIER SEQUOIA S.A., LAUSANNE, CH, vol. 55, no. 1, 1999, pages 77-89, XP002228955 ISSN: 0925-4005**
• **FANIGLIULO A ET AL: "Comparison between a LAPS and an FET-based sensor for cell-metabolism detection" SENSORS AND ACTUATORS B, ELSEVIER SEQUOIA S.A., LAUSANNE, CH, vol. 32, no. 1, 1 April 1996 (1996-04-01), pages 41-48, XP004053954 ISSN: 0925-4005**
• **SOHN B-K ET AL: "A NEW PH-ISFET BASED DISSOLVED OXYGEN SENSOR BY EMPLOYING ELECTROLYSIS OF OXYGEN" SENSORS AND ACTUATORS B, ELSEVIER SEQUOIA S.A., LAUSANNE, CH, vol. 34, no. 1/3, 1 August 1996 (1996-08-01), pages 435-440, XP000678112 ISSN: 0925-4005**
• **WOLF B ET AL: "Biofunctional hybrid structures-cell-silicon hybrids for application in biomedicine and bioinformatics" BIOELECTROCHEMISTRY AND BIOENERGETICS, vol. 46, 1998, pages 215-225, XP002265839**

## Beschreibung

Die Erfindung betrifft ein Verfahren nach dem Oberbegriffvon Anspruch 1.

Ein derartiges Verfahren ist aus "Biofunctional hybrid structures - cell-silicon hybrids for applications in biomedicine and bioinformatics", Wolf, B. et. al., Biochemistry and Biogenergetics 46 (1998), Seiten 215-225 bekannt. Dabei werden lebende biologische Zellen in einer von einem Kulturmedium durchflossen Messkammer angeordnet, in deren Boden ein ionenselektiver Feldeffekt-Transostore (ISFET) und ein Dünnfilm-Sauerstoffsensor integriert sind. Mit Hilfe des ISFET wird ein pH-Wertmessignal erfasst, um die von den Zellen bewirkte Ansäuerung des Kulturmediums zu bestimmen. Mittels des Sauerstoffsensors wird ein Messsignal für die Atmung der Zellen aufgezeichnet. Bei diesem Verfahren werden die Messwerte für pH-Wert und Sauerstoffgehalt an unterschiedlichen Stellen und somit an unterschiedlichen, in der Messkammer befindlichen biologischen Zellen gemessen. Zellkulturen, Zellschnitte, Gewebe und dergleichen zu untersuchendes biologisches Material sind jedoch oft heterogen, d.h. unterschiedliche Zellen können unterschiedliche Signale abgeben. Bei dem Verfahren kann es deshalb zu Ungenauigkeiten bei der Untersuchung eines in dem Kulturmedium enthaltenen Wirkstoffs kommen, wenn der pH-Wert und/oder der Sauerstoffgehalt an den Messorten der Sensoren für pH-Wert und Sauerstoffgehalt voneinander abweichen.

Aus EP 0 394 406 B1 ist ferner ein Verfahren bekannt, bei dem lebende biologische Zellen in einer von einem Kulturmedium durchflossen Mikrofließkammer angeordnet werden, die an ihrem Boden einen Silizium-Sensor aufweist. In der Mikrofließkammer ist eine Referenzelektrode vorgesehen, die mit dem Kulturmedium in Kontakt steht. Bei dem vorbekannten Verfahren wird in einem ersten Verfahrensschritt der Durchfluß des Kulturmediums in der Mikroftießkammer gestoppt. Dann wird die pH-Wertänderung, als metabolischer Stoffwechselindikator in dem in der Mikrofließkammer befindlichen Kulturmedium durch Messung des elektrischen Potentials zwischen dem Silizium-Sensor und der Referenzelektrode gemessen. Mit dem Verfahren lässt sich z.B. die Wirkung eines in dem Kulturmedium enthaltenen zellbeeinflussenden Wirkstoffs auf den Stoffwechsel der Zellen messen. Ungünstig ist dabei jedoch, dass das Verfahren nur eine Aussage über die Ausscheidung saurer Stoffwechselprodukte in Form einer pH-Wertänderung in dem Kulturmedium ermöglicht, während andere, durch den Stoffwechsel der Zellen verursachte Veränderungen nicht berücksichtigt werden.

Aus DE 44 17 078 A1 kennt man auch bereits ein Verfahren, bei dem in einer Mikrofließkammer befindliche biologische Zellen mittels mehrerer unterschiedlicher Mikrosensoren gleichzeitig untersucht werden. Dabei wird zusätzlich zu der Ansäuerung gleichzeitig auch noch die Atmung der Zellen in dem Kulturmedium überwacht, was eine genauere Untersuchung der Wirkung eines in dem Kulturmedium enthaltenen Wirkstoffs auf die Zellen ermöglicht. Auch bei diesem Verfahren werden die Messwerte für pH-Wert und Sauerstoffgehalt an unterschiedlichen Messorten gemessen. Wenn der pH-Wert und/oder der Sauerstoffgehalt an den Messorten voneinander abweichen, können Messfehler auftreten.

Es besteht deshalb die Aufgabe, ein Verfahren zur Untersuchung von membranumschlossenen Biokompartimenten anzugeben, das es ermöglicht, an einem Ort mehrere Stoffwechselparameter der Biokompartimente zu messen.

Diese Aufgabe wird mit den Merkmalen des Anspruchs 1 gelöst.

Die Lösung dieser Aufgabe besteht insbesondere darin,

a) dass zum indirekten Messen der Konzentration eines von den Biokompartimenten (1) beim Stoffwechsel abgebbaren und/oder aufnehmbaren Stoffes in einem im Wirkungsbereich der Biokompartimente (1) befindlichen Teilbereich des Kulturmediums eine Arbeitselektrode (19) und eine dazu beabstandete Referenzelektrode (20) angeordnet werden, an die eine elektrische Spannung anlegbar ist,

b) dass bei abgeschalteter elektrischer Spannung oder bei einer Spannung, bei der die Bildung von Hydroxid- und Wasserstoff-Ionen aus dem in dem Kulturmedium befindlichen Stoff unterbleibt, zumindest ein erster Messwert ($pH_{-1}$, $pH_{-3}$ bzw. $pH_{+1}$, $pH_{+3}$) für den pH-Wert des Kulturmediums gemessen wird,

c) dass nach der Messung des ersten Messwertes ($pH_{-1}$, $pH_{-3}$ bzw. $pH_{+1}$, $pH_{+3}$) eine elektrische Spannung derart an das Kulturmedium angelegt wird, dass aus dem in dem Kulturmedium befindlichen Stoff Hydroxid-Ionen oder Wasserstoff-Ionen gebildet werden,

d) dass In dem Teilbereich des Kulturmediums befindlichen Messstelle während des Anliegens dieser elektrischen Spannung wenigstens ein zweiter Messwert ($pH_{-2}$, $pH_{-4}$ bzw. $pH_{+2}$, $pH_{+4}$) für den pH-Wert des Kulturmediums gemessen wird,

e) dass aus dem ersten Messwert ($pH_{-1}$, $pH_{-3}$ bzw. $pH_{+1}$, $pH_{+3}$) und dem zweiten Messwert ($pH_{-2}$, $pH_{-4}$ bzw. $pH_{+2}$, $pH_{+4}$) die Differenz ($\Delta pH_{-21}$, $\Delta pH_{-43}$ bzw. $\Delta pH_{+21}$, $\Delta pH_{+43}$) gebildet wird,

f) dass die Schritte a) bis e) wenigstens einmal wiederholt werden,

g) dass aus dem Unterschied zwischen wenigstens zwei dieser Messwert-Differenzen ($\Delta pH_{-21}$, $\Delta pH_{-43}$ bzw. $\Delta pH_{+21}$, $\Delta pH_{+43}$) die Konzentrationsänderung des Stoffs in dem Kulturmedium und aus dem Unterschied zwischen wenigstens zwei der ersten pH-Messwerte ($pH_{-1}$, $pH_{-3}$ bzw. $pH_{+1}$, $pH_{+3}$) und/oder zweiten pH-Messwerte ($pH_{-2}$, $pH_{-4}$ bzw. $pH_{+2}$, $pH_{+4}$) die Ansäuerung oder Basifizierung des Kulturmediums ermittelt werden,

h) und dass aus den so gewonnenen Messwerten für die Konzentrafionsänderung und die Ansäuerung oder Basifizierung die Stoffwechselaktivität der Biokompartimente(1) bestimmt wird.

Das Verfahren beruht auf der Erkenntnis, daß die Biokompartimente den pH-Wert des Kulturmediums kontinuierlich mit etwa konstanter Steigung verändern und daß diese pH-Wert-Änderung wesentlich langsamer verläuft, als die während des Anlegens der elektrischen Spannung zur Bildung von Hydroxid-Ionen (OH⁻-Ionen) oder Wasserstoff-Ionen (H⁺-Ionen) aus dem in dem Kulturmedium enthaltenen, beim Stoffwechsel von den Biokompartimenten abgebbaren und/oder aufnehmbaren Stoff bewirkte pH-Wert-Änderung. In vorteilhafter Weise ist es dadurch möglich, trotz der Überlagerung der von den Biokompartimenten in das Kulturmedium abgegebenen oder aus diesen aufgenommenen Hydroxid- und/oder Wasserstoffionen mit den aus den in dem Kulturmedium enthaltenen Stoff gebildeten Hydroxid- und/oder Wasserstoffionen an ein und derselben Meßstelle und mit nur einem einzigen pH-Sensor sowohl die durch die Biokompartimente bewirkte Konzentrationsänderung des von den Biokompartimenten abgebbaren und/oder aufnehmbaren Stoffs als auch die durch die Biokompartimente bewirkte pH-Wert-Änderung in dem Kulturmedium zu messen. Mit dem Verfahren können insbesondere die Atmung der Biokompartimente durch Messung der Sauerstoffkonzentrationsänderung sowie die Konzentrationsänderung von Stickstoffmonoxid (NO), Wasserstoffperoxid ($H_2O_2$) und/oder andere Sauerstoff und/oder Wasserstoff in chemisch gebundener Form enthaltende Stoffe, wie z.B. auch sich an der Zelloberfläche befindende Moleküle, gemessen werden. Eine durch Unterschiede der einzelnen Zellen der Zellkultur bewirkte Ortsabhängigkeit der Konzentrationsänderung und/oder der pH-Wertänderung wirkt sich daher auf die Genauigkeit der Bestimmung des Stoffwechsels der zu untersuchenden Zellen praktisch nicht aus.

Aus Sohn, B.-H., Kim, C.-S., "A new pH-ISFET based dissolved oxygen sensor by employing electrolysis of oxygen" in Sensors and Actuators B34 (1996), Seite 435-440 ist zwar bereits ein gattungsfremdes Verfahren bekannt, bei dem der Sauerstoffgehalt in einem Analyten indirekt über den pH-Wert gemessen wird, wobei an dem Analyten das elektrische Potential soweit in den negativen Bereich abgesenkt wird, daß aus dem in dem Analyten befindlichen Sauerstoff Hydroxid-Ionen gebildet werden, und wobei diese Hydroxid-Ionen mittels eines ionenselektiven Sensors detektiert werden. Der Analyt enthält jedoch keine Biokompartimente, die während der Messung z.B. durch saure Stoffwechselprodukte und Sauerstoffaufnahme den Analyten verändern. Auch erfolgt bei dem vorbekannten Verfahren keine Auswertung des Sensorsignals hinsichtlich der Bestimmung der Atmungsaktivität und der Ansäuerung bei nicht abgesenktem elektrischen Potential.

Bei einer besonders vorteilhaften Ausführungsform des Verfahrens ist vorgesehen, daß die Abfolge bestehend aus den Schritten a) bis g) wenigstens zweimal durchlaufen wird und daß bei diesen Durchläufen die Polarität der zwischen der Arbeitselektrode und der Referenzelektrode angelegten elektrischen Spannung unterschiedlich gewählt wird. Dadurch ist es möglich, mit nur einem pH-Wertsensor sowohl die Konzentrationsänderung von elektronegativen Stoffen, wie z.B. Sauerstoff, als auch die Konzentration von elektropositiven Stoffen, wie z.B. Stickstoffmonoxid oder Wasserstoffperoxid, in dem Kulturmedium zu messen. Dabei wird zur Messung der Konzentrationsänderung eines elektronegativen Stoffs ein gegenüber dem Potential der Referenzelektrode negatives elektrisches Potential an der Bezugselektrode angelegt, während das Bezugselektrodenpotential zur Messung der Konzentrationsänderung eines elektropositiven Stoffs positiv gewählt wird.

Vorteilhaft ist, wenn die Zeitpunkte für die Messung der pH-Meßwerte so auf die Zeitpunkte, an denen das elektrische Potential verändert oder abgeschaltet wird, abgestimmt werden, daß sich die Wasserstoff- und Hydroxid-Ionenkonzentration in dem Kulturmedium zu den Meßzeitpunkten im wesentlichen im Gleichgewicht befindet. Der Stoffwechsel bzw. die Vitalität der Biokompartimente kann dadurch mit großer Präzision gemessen werden.

Bei einer vorteilhaften Ausführungsform der Erfindung ist vorgesehen, dass jeweils der Zeitabstand zwischen dem Zeitpunkt des Anlegens, Abschaltens oder Veränderns der elektrischen Spannung und dem darauffolgenden Zeitpunkt, in dem ein pH-Messwert gemessen wird, bei allen pH-Messwerten etwa gleich ist. Dabei wird durch die Bildung der Differenz aus den ersten und zweiten pH-Messwerten bei der Messung der Konzentrationsänderung des am Stoffwechsel der Biokompartimente beteiligten Stoffs in dem Kulturmedium beziehungsweise aus den ersten pH-Messwerten bei der Messung der durch die Biokompartimente verursachten pH-Wertänderung des Kulturmediums der Einfluss, den der zeitliche Abstand zwischen dem Zeitpunkt des Beginns einer Potentialveränderung und der darauffolgenden Messung des pH-Messwertes hat, kompensiert. Somit ergibt sich eine noch größere Messgenauigkeit.

Besonders vorteilhaft ist, wenn die elektrische Spannung abgeschaltet oder gemäß Schritt b) angelegt wird, bevor sich ein Gleichgewichtszustand der Wasserstoff- und Hydroxid-Ionenkonzentration in dem Kulturmedium ausgebildet hat. Die in der Mikrofließkammer befindlichen Biokompartimente werden dann jeweils nur kurz mit dem zur Bildung der Hydroxid- oder Wasserstoffionen geeigneten elektrischen Potential beaufschlagt und bleiben somit durch das Anlegen des Potentials in ihrem physiologischen Zustand weitgehend unbeeinflusst. Dadurch ist es möglich, über einen längeren Zeitraum Messungen an den Biokompartimenten durchzuführen, beispielsweise um mehrere unterschiedliche Wirkstoffe nacheinander mit den Biokompartimenten in Berührung zu bringen und deren Wirkung auf die Biokomparfimente miteinander zu vergleichen.

Bei einer vorteilhaften Ausführungsform des Verfahrens wird der zwischen der Arbeitselektrode und der Referenzelektrode fließende elektrische Elektrodenstrom gemessen. Der Verlauf des Elektrodenstroms kann dann mit dem Verlauf der an die Elektroden angelegten elektrischen Spannung und ggf. einer zuvor abgespeicherten Strom-Spannungskennlinie oder einer -kennlinienschar verglichen werden, um die Plausibilität der Messergebnisse zu überprüfen.

Zweckmäßigerweise werden die Biokompartimente derart an der Arbeitselektrode angelagert, daß sie diese zumindest bereichsweise überdecken. Aus den zeitlichen Verläufen der zwischen Arbeitselektrode und Bezugselektrode anliegenden elektrischen Spannung und dem Elektrodenstrom kann dann eine eventuelle Veränderung der Morphologie oder Adhäsion der Biokompartimente bestimmt werden.

Besonders vorteilhaft ist, wenn die pH-Meßwerte mittels eines ionenselektiven Feldeffekttransistors (ISFET) gemessen werden. Das Verfahren ermöglicht dann wegen der kleinen Abmessungen eines solchen Sensors eine hohe Ortsauflösung, wobei es sogar möglich ist, einzelne Zellen der Zellkultur zu untersuchen und die dabei erhaltenen Meßwerte miteinander zu vergleichen. Auf diese Weise können beispielsweise in einer Zellkultur einzelne Zellen, die auf einen bestimmten Wirkstoff besonders empfindlich reagieren, lokalisiert werden.

Bei einer besonders vorteilhaften Ausführungsform der Erfindung wird der Durchfluß des Kulturmediums während und zwischen der Erfassung des ersten und letzten pH-Meßwertes einer zur Bestimmung der Atmungsaktivität der Biokompartimente und der durch die Biokompartimente bewirkten pH-Wertänderung des Kulturmediums vorgesehenen pH-Wert-Messreihe angehalten oder zumindest reduziert. Der Sauerstoffgehalt nimmt dann nach dem Anhalten oder Reduzieren des Kulturmedium-Durchflusses bei Sauerstoff aufnehmenden Biokompartimenten ab und bei durch Photosynthese Sauerstoff produzierenden Biokompartimenten zu. Somit ergibt sich jeweils ein entsprechend großes Meßsignal, was eine genaue Bestimmung der Atmungsaktivität der Biokompartimente ermöglicht. Bei Biokompartimenten, die Sauerstoff aufnehmen, kann nach dem Anhalten oder Reduzieren des Kulturmedium-Durchflusses eine Zunahme des pH-Wertes des Kulturmediums und bei Biokompartimenten, die Sauerstoff produzieren, eine Abnahme des pH-Wertes beobachtet werden. Somit ergibt sich auch bei der durch die Biokompartimente bewirkten pH-Wert-Änderung des Kulturmediums ein entsprechend großes Meßsignal.

Nachfolgend sind Ausführungsbeispiele der Erfindung anhand der Zeichnung näher erläutert. Es zeigen zum Teil stärker schematisiert:

Fig. 1 einen Längsschnitt durch eine Vorrichtung mit einer Mikrofließkammer zur Untersuchung von lebenden, membranumschlossenen Biokompartimenten,

Fig. 2 einen Längsschnitt durch einen in den Boden der Mikrofließkammer eingelassenen ISFET mit einer benachbart dazu angeordneten Elektrode, wobei an dem ISFET und der Elektrode biologische Zellen adhärent angelagert sind,

Fig. 3 eine Aufsicht auf die in Fig.2 gezeigte Anordnung,

Fig. 4 eine graphische Darstellung eines mittels eines ISFETs gemessenen Meßsignales, wobei der Meßsignalverlauf nur schematisch dargestellt ist,

Fig. 5 eine graphische Darstellung eines mittels eines ISFETs in der Mikrofließkammer gemessenen Meßsignales, wobei auf der Abszisse die Zeit und auf der Ordinate der pH-Wert aufgetragen ist und wobei das in der Mikrofließkammer befindliche Zell-Kulturmedium weitestgehend frei von zellbeeinflussenden Wirkstoffen ist,

Fig. 6 eine Darstellung ähnlich Fig.5, wobei jedoch in dem Zell-Kulturmedium Jodacetat als zellbeeinflussender Wirkstoff enthalten ist,

Fig. 7 eine Darstellung ähnlich Fig.5, wobei jedoch in dem Zell-Kulturmedium Triton® enthalten ist und

Fig. 8 eine Darstellung ähnlich Fig.4, wobei jedoch bei jedem Anlegen einer elektrischen Spannung an das Kulturmedium die Polarität der Spannung verändert wird.

Bei einem Verfahren zur Untersuchung von membranumschlossenen Biokompartimenten werden als biologische Zellen ausgebildete Biokompartimente 1 in einer Mikrofließkammer angeordnet. Die Mikrofließkammer 2 weist eine von einem Kammergehäuse umgrenzte Meßkammer 3 mit einer Einlaßöffnung 4 und einer Auslaßöffnung 5 für ein Nähr- oder Kulturmedium auf. Das Kammergehäuse hat ein Kammerunterteil 6 und ein dazu passendes Deckelteil 7, die durch einen dazwischen befindlichen Dichtring 8 flüssigkeitsdicht miteinander verbunden sind.

Die Einlaßöffnung 4 der Meßkammer 3 ist über einen Zuführkanal 9 mit einem Kulturmedium-Vorrat 10 verbunden. In dem Zuführkanal 9 ist eine Pumpe 11 zum Fördern des Kulturmediums angeordnet. Die Pumpe 11 ist mittels einer in der Zeichnung nicht dargestellten Steuereinrichtung ein- und ausschaltbar. Die Auslaßöffnung 5 der Mikrofließkammer 2 ist über eine Ableitung 12 an einer Austrittsöffnung 13 für das Kulturmedium angeschlossen. In der Meßkammer 3 stehen die Biokompartimente 1 mit dem Kulturmedium in Berührung.

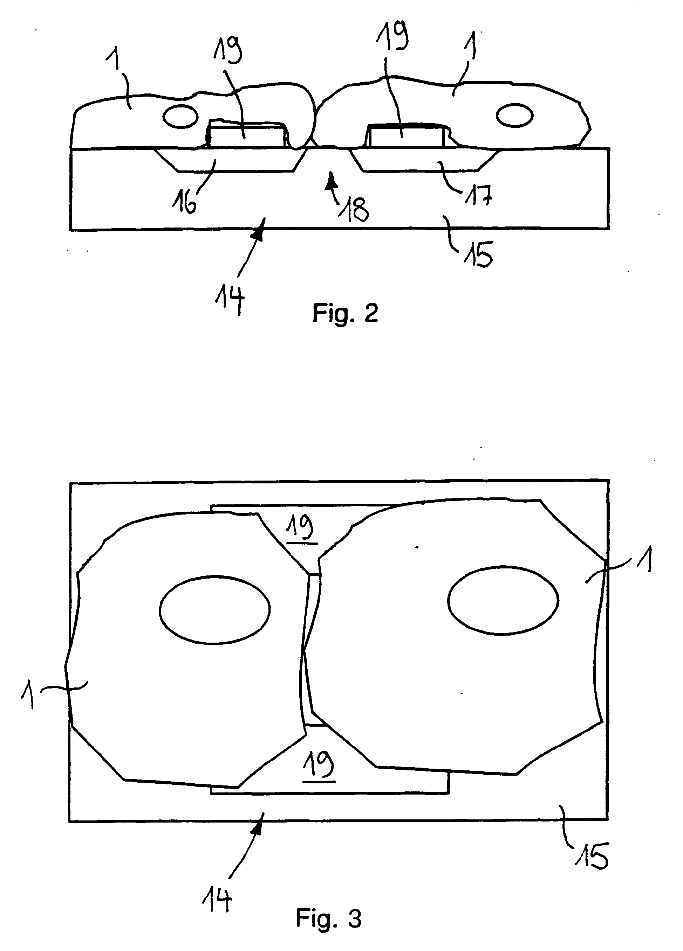

Im Boden der Meßkammer 3 sind mehrere ionenselektive Feldeffekttransistoren (ISFETs) 14 angeordnet. Wie in Fig.2 erkennbar ist, weisen die ISFETs ein aus einem Halbleitermaterial bestehendes Substrat 15 auf, in das für Drain 16 und Source 17 hochdotierte Zonen mit zum Substrat 15 entgegengesetzten Leitungstyp angeordnet sind. Zwischen Drain 16 und Source 17 ist das Gate 18 angeordnet. Gate 18, Drain 16 und Source 17 sind über eine darüber befindliche, in der Zeichnung nicht näher dargestellte Silizium-Nitrid-Schicht gegen die Meßkammer 3 elektrisch isoliert. Die Silizium-Nitrid-Schicht bildet an ihrer dem Gate 18 abgewandten Außenseite eine aktive Sensorschicht, über die der ISFET 14 mittels dort in dem Kulturmedium befindlicher Wasserstoff-Ionen steuerbar ist. Gate 18, Drain 16 und Source 17 der ISFETs 14 sind mit einer an sich bekannten Auswerteeinrichtung zur Messung des zu dem Meßsignal des jeweiligen ISFETs 14 etwa proportionalen pH-Wert des Kulturmediums verbunden.

Wie in Fig.2 besonders gut erkennbar ist, ist dicht benachbart zu den Gates 18 jeweils eine aus einem Edelmetall, wie z.B. Palladium, bestehende Arbeitselektrode 19 angeordnet, die mit dem Kulturmedium in Kontakt steht. Bei dem Ausführungsbeispiel nach Fig. 2 und 3 ist die Arbeitselektrode 19 auf der Drain 16, Source 17 und Gate 18 überdeckenden Siliziumnitrid-Schicht angeordnet und umgrenzt das Gate 18 ringförmig.

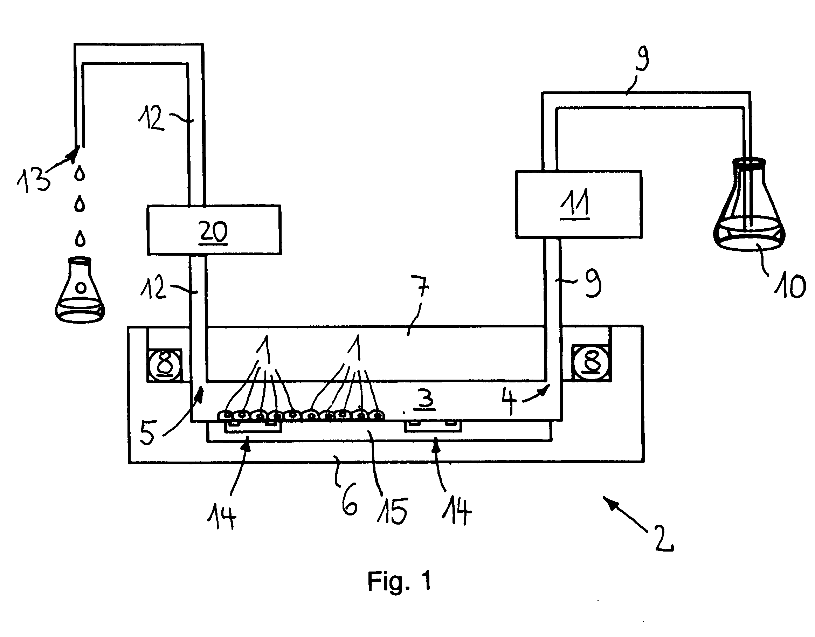

Den Arbeitselektroden 19 ist eine Referenzelektrode 20 zugeordnet, die das Kulturmedium mit Abstand zu den ISFETs 14 kontaktiert. Bei dem Ausführungsbeispiel nach Fig. 1 ist diese Referenzelektrode 20 in der Ableitung 12 für das Kulturmedium angeordnet. Zum Anlegen eines elektrischen Potentials an das Kulturmedium sind die Referenzelektrode 20 und die Arbeitselektroden 19 mit einer elektrischen Spannungsquelle verbindbar.

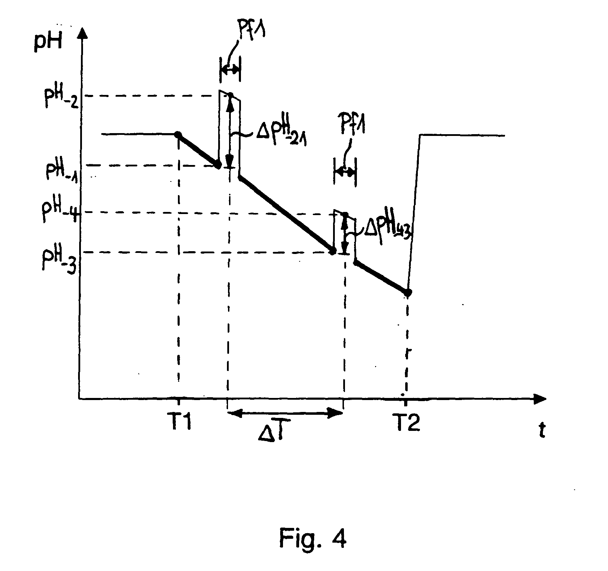

Mittels der vorstehend beschriebenen Meßanordnung läßt sich die Atmungsaktivität der Biokompartimente 1 indirekt messen. Dazu wird die Pumpe 11, welche den Durchfluß des Kulturmediums durch die Messkammer 3 bewirkt, vorübergehend angehalten. In Fig.4 ist das Zeitintervall, während dem der Durchfluß in der Meßkammer 3 gestoppt ist, durch die Zeitpunkte T1 (Abschalten der Pumpe 11) und T2 (Einschalten der Pumpe 11) markiert. Dieses Zeitintervall kann beispielsweise 8 Minuten betragen.

Danach wird mittels des ISFETs 14 für den pH-Wert des Kulturmediums ein erster Meßwert $pH_{-1}$ für ein erstes pH-Meßwertpaar gemessen. Dann wird in einem im Wirkungsbereich der Biokompartimente 1 befindlichen Teilbereich des Kulturmediums das elektrische Potential durch Anlegen einer elektrischen Spannung zwischen Referenzelektrode 20 und Arbeitselektrode 19 an der Arbeitselektrode 19 soweit in den negativen Bereich abgesenkt, daß aus in dem Kulturmedium befindlichem Sauerstoff Hydroxid-Ionen gebildet werden. Dabei beträgt dieses Potential vorzugsweise etwa -700 bis -800 Millivolt. Bei dem Ausführungsbeispiel nach Fig.3 wird dieses Potential etwa 30 Sekunden an die Elektroden 19, 20 angelegt. Die mit dem Kulturmedium in Kontakt stehende Oberfläche der Arbeitselektrode 19 wird möglichst klein gewählt und beträgt vorzugsweise weniger als 1000 $\mu m^2$, um bei angelegter elektrischer Spannung den Sauerstoffverbrauch an der Arbeitselektrode 19 möglichst gering zu halten. In Fig.4 ist das Zeitintervall, an dem das negative Potential an die Arbeitselektrode 19 angelegt wird, jeweils durch einen Doppelpfeil Pf1 markiert. Während des Anlegens des negativen elektrischen Potentials wird im Bereich der Arbeitselektrode 19 in dem Kulturmedium befindlicher Sauerstoff entsprechend den nachfolgenden Reaktionsgleichungen reduziert:

$$O_2 + 2H_2O + 2e^- \rightarrow H_2O_2 + 2OH^-$$

$$H_2O_2 + 2e^- \rightarrow 2OH^-$$

und

$$O_2 + 2H_2O_2 + 4e^- \rightarrow 4OH^-$$

Die dabei an der Arbeitselektrode 19 gebildeten Hydroxid-Ionen bewirken in dem Kulturmedium eine lokale Veränderung des pH-Wertes, die mittels des ISFETs 14 meßbar ist. In Fig.4 ist deutlich erkennbar, daß der pH-Wert während des Anlegens des negativen elektrischen Potentials an die Arbeitselektrode 19 um einen dem Sauerstoffgehalt des Kulturmediums entsprechenden Wert ansteigt. Da die an der Arbeitselektrode 19 gebildeten Hydroxid-Ionen von der Arbeitselektrode 19 wegdiffundieren und einen Konzentrationsgradienten bilden, wird die Arbeitselektrode 19 möglichst dicht an dem Gate 18 des ISFET 14 angeordnet, um bei der Detektion dieser Hydroxid-Ionen eine möglichst große Empfindlichkeit des ISFET 14 zu erreichen.

Bei an der Arbeitselektrode 19 anliegendem negativen Potential wird mittels des ISFET 14 ein zweiter pH-Meßwert $pH_{-2}$ für das erste pH-Meßwertpaar in dem Kulturmedium gemessen. Danach wird das elektrische Potential soweit angehoben, daß die Bildung von Hydroxid-Ionen aus dem in dem Kulturmedium befindlichen Sauerstoff unterbleibt, beispielsweise auf einen Wert zwischen Null und +800 Millivolt. In Fig.4 ist deutlich erkennbar, daß der pH-Wert mit dem Zurücknehmen der Potentialabsenkung abnimmt.

Dann wird mittels des ISFET 14 für den pH-Wert des Kulturmediums ein erster Meßwert $pH_{-3}$ für ein zweites pH-Meßwertpaar gemessen und danach wird das Potential an der Arbeitselektrode 19 erneut soweit in den negativen Bereich abgesenkt, daß aus in dem Kulturmedium befindlichem Sauerstoff Hydroxid-Ionen gebildet werden. In Fig.4 ist deutlich der entsprechende Anstieg des Meßsignales des ISFETs erkennbar. Nun wird ein zweiter pH-Meßwert $pH_{-4}$ des zweiten pH-Meßwertpaares mittels des ISFETs erfasst und danach wird das elektrische Potential soweit angehoben, daß die Bildung von Hydroxid-Ionen aus dem in dem Kulturmedium befindlichen Sauerstoff unterbleibt. Außerdem wird die Pumpe 11 wieder zugeschaltet.

Aus zeitlich zueinander benachbarten, einander zugeordneten ersten und zweiten pH-Meßwerten der beiden pH-Meßwertpaare wird jeweils ein pH-Differenzwert

$$\Delta pH_{-21} = pH_{-2} - pH_{-1}$$

$$\Delta pH_{-43} = pH_{-4} - pH_{-3},$$

gebildet der ein Maß für den Sauerstoffgehalt in dem Kulturmedium darstellt. Aus dem Unterschied ($\Delta pH_{-43} - \Delta pH_{-21}$) dieser zeitlich aufeinanderfolgenden Differenzwerte $pH_{-43}$, $pH_{-21}$ und dem Abstand $\Delta T$ zwischen den Zeitpunkten, an denen die pH-Differenzwerte $pH_{-43}$, $pH_{-21}$ gemessen wurden, wird die Atmungsaktivität oder Atmungsleistung

$$\frac{\Delta pH_{-43} - \Delta pH_{-21}}{\Delta T}$$

der Biokompartimente 1 bestimmt.

Zusätzlich wird aus dem Unterschied der ersten pH-Meßwerte $pH_{-1}$, $pH_{-3}$ die Ansäuerung

$$\Delta pH_{-31} = pH_{-3} - pH_{-1}$$

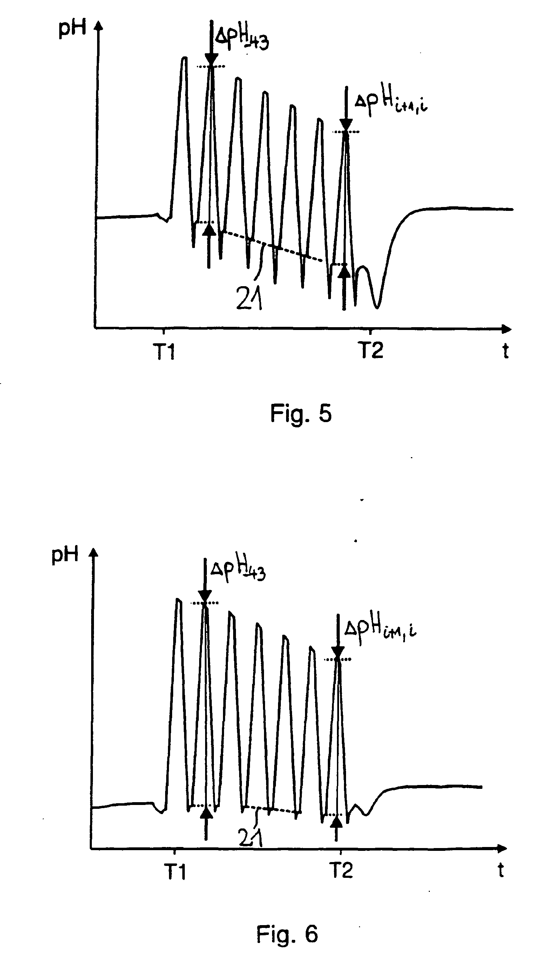

ermittelt. Bei den in Fig. 4 bis 6 gezeigten Ausführungsbeispielen ist diese pH-Wert-Änderung negativ, d.h. die Biokompartimente säuern während der Stillstandsphase der Pumpe 11 das Kulturmedium an. In Fig.5 und 6 ist diese Ansäuerung jeweils durch einen Geradenabschnitt 21 strichliniert dargestellt. Aus den so gewonnen Meßwerten für Atmungsaktivität und Ansäuerung wird auf den Stoffwechsel der Biokompartimente 1 geschlossen. Entsprechend kann bei photosynthetisch aktiven Biokompartimenten 1, wie z.B. Algen, die Sauerstoffabgabe und Basifizierung gemessen werden.

Die aus dem Absenken und Anheben des elektrischen Potentials an der Arbeitselektrode 19 sowie dem Erfassen jeweils eines ersten pH-Meßwerts $pH_{-1}$, $pH_{-3}$ und jeweils eines zweiten pH-Meßwertes $pH_{-2}$, $pH_{-4}$ bestehenden Verfahrensschritte können gegebenenfalls auch mehrmals während der Stillstandphase der Pumpe 11 wiederholt werden, wie dies bei den Ausführungsbeispielen gemäß Fig.5 bis 7 erkennbar ist. In Fig. 5 bis 7 ist der bei dem i-ten Wiederholen dieser Verfahrensschritte ermittelte pH-Differenzwert jeweils mit $\Delta pH_{i+1,i}$ bezeichnet.

Bei dem Ausführungsbeispiel nach Fig.5 ist das Kulturmedium im wesentlichen frei von zellbeeinflussenden Wirkstoffen. Während der durch die Zeitpunkte T1 und T2 markierten Stillstandsphase der Pumpe 11 ist eine deutliche Ansäuerung des Kulturmediums durch die Biokompartimente 1 erkennbar. Wie aus dem Unterschied der pH-Differenzwerte $\Delta pH_{-43}$ und $\Delta pH_{i+1,i}$ erkennbar ist, nehmen die Biokompartimente Sauerstoff aus dem Kulturmedium auf.

Bei dem Ausführungsbeispiel nach Fig.6 enthält das Kulturmedium Jodacetat als zellbeeinflussenden Wirkstoff. Durch einen Vergleich mit Fig.5 ist erkennbar, daß die durch die Biokompartimente 1 bewirkte Ansäuerung $\Delta pH_{-31}$ des Kulturmediums abnimmt. Das Jodacetat hindert die Biokompartimente 1 weitestgehend daran, Wasserstoffionen an das Kulturmedium abzugeben. Im Vergleich zu Fig.5 ist außerdem erkennbar, daß das Jodacetat eine Erhöhung der Atmungsaktivität der Biokompartimente 1 bewirkt.

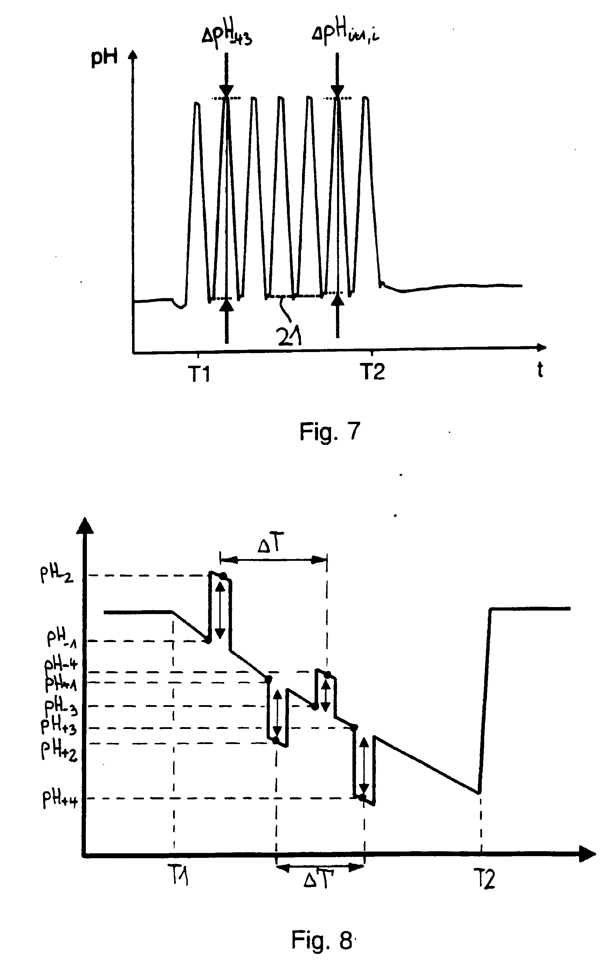

Bei dem Ausführungsbeispiel nach Fig.7 enthält das Kulturmedium das Detergenz Triton®, das zum Absterben der Biokompartimente 1 führt. Deutlich ist erkennbar, daß die Biokompartimente 1 nach dem Kontakt mit dem Triton® wegen Auflösung der mitochondrialen Atmungsketten keinerlei Atmungsaktivität mehr aufweisen und auch den pH-Wert des Kulturmediums nicht mehr beeinflussen.

In Fig. 5 bis 7 ist noch erkennbar, daß die Zeitpunkte für die Messung der ersten und zweiten pH-Meßwerte so auf die Zeitpunkte, an denen das elektrische Potential an der Arbeitselektrode abgesenkt oder angehoben wird, abgestimmt werden, daß die Bildungsrate der Hydroxid-Ionen zu den Meßzeitpunkten im wesentlichen der Bildungsrate der Hydroxid-Ionen im Zeitpunkt kurz vor dem Absenken oder Anheben der elektrischen Spannung entspricht. Die Hydroxid-Ionenkonzentration befindet sich somit zu den Meßzeitpunkten im wesentlichen im Fließgleichgewicht. In Fig. 5 bis 7 sind einige der ersten beziehungsweise zweiten pH-Meßwerte jeweils durch horizontale, strichpunktierte Linien markiert.

Bei dem Ausführungsbeispiel nach Figur 8 wird nach dem Abschalten der Pumpe 11 zunächst ein erster Meßwert $pH_{-1}$ und ein zweiter $pH_{-2}$ Meßwert für ein erstes pH-Meßwertpaar einer Stickstoffmonoxid-Konzentrationsmessung gemessen, wobei wie bei dem Ausführungsbeispiel nach Figur 3 vorgegangen wird. Danach wird die elektrische Spannung abgeschaltet. Dann wird mittels des ISFETs 14 für den pH-Wert des Kulturmediums ein erster Meßwert $pH_{+1}$ für ein erstes pH-Meßwertpaar einer Stickstoffmonoxid-Konzentrationsmessung gemessen. Da das von den Biokompartimenten 1 gebildete Stickstoffmonoxid in dem Kulturmedium nur eine geringe Halbwertszeit aufweist, ist der ISFET 14 vorzugsweise direkt an der Zellmembran oder dicht benachbart dazu angeordnet. Dann wird in dem im Wirkungsbereich der Biokompartimente 1 befindlichen Teilbereich des Kulturmediums das elektrische Potential durch Anlegen einer elektrischen Spannung zwischen Referenzelektrode 20 und Arbeitselektrode 19 an der Arbeitselektrode 19 soweit in den positiven Bereich angehoben, daß aus dem im Kulturmedium befindlichen Stickstoffmonoxid (NO) Wasserstoff-Ionen gebildet werden. Dabei beträgt dieses Potential vorzugsweise etwa +700 bis +800 Millivolt, bezogen auf das Potential der Referenzelektrode 20. Dann wird mittels des ISFET 14 ein zweiter pH-Meßwert $pH_{+2}$ für das erste pH-Meßwertpaar der Stickstoffmonoxid-Konzentrationsmessung in dem Kulturmedium gemessen.

In Fig.8 sind die Zeitintervalle, in denen die positive elektrische Spannung an die Elektroden 19, 20 angelegt wird, jeweils durch den Doppelpfeil Pf1 und die Zeitintervalle, in denen die negative elektrische Spannung an die Elektroden 19, 20 angelegt wird, jeweils durch den Doppelpfeil Pf2 markiert. Während des Anlegens des positiven elektrischen Potentials wird im Bereich der Arbeitselektrode 19 in dem Kulturmedium befindliches Stickstoffmonoxid entsprechend der Reaktionsgleichung

$$NO + H_2O - e^- \rightarrow NO_2^- + 2H^+$$

reduziert. Die dabei an der Arbeitselektrode 19 gebildeten Wasserstoff-Ionen bewirken in dem Kulturmedium eine lokale Veränderung des pH-Wertes, die mittels des ISFETs 14 meßbar ist. In Fig.8 ist deutlich erkennbar, daß der pH-Wert während des Anlegens des positiven elektrischen Potentials an die Arbeitselektrode 19 um einen dem Stickstoffmonoxidgehalt des Kulturmediums entsprechenden Wert abfällt.

Dann wird die elektrische Spannung erneut abgeschaltet und es wird ein erster Meßwert $pH_{-3}$ für ein zweites pH-Meßwertpaar der Sauerstoff-Konzentrationsmessung ermittelt. Danach wird das Potential an der Arbeitselektrode 19 soweit in den negativen Bereich abgesenkt, daß aus in dem Kulturmedium befindlichem Sauerstoff Hydroxid-Ionen gebildet werden. Nun wird ein zweiter pH-Meßwert $pH_{-4}$ des zweiten pH-Meßwertpaares der Sauerstoff-Konzentrationsmessung mittels des ISFETs erfasst. Dann wird die elektrische Spannung abgeschaltet und es wird ein erster Meßwert $pH_{+3}$ für ein zweites pH-Meßwertpaar der Stickstoffmonoxid-Konzentrationsmessung gemessen. Nachdem das elektrische Potential erneut soweit angehoben wurde, daß aus dem in dem Kulturmedium befindlichen Stickstoffmonoxid Wasserstoff-Ionen gebildet werden, wird ein zweiter pH-Meßwert $pH_{+4}$ des zweiten pH-Meßwertpaares der Stickstoffmonoxid-Konzentrationsmessung mittels des ISFETs erfasst und die Pumpe 11 wird wieder eingeschaltet.

Aus den pH-Differenzwerten

$$\Delta pH_{-21} \; = \; pH_{-2} \; - \; pH_{-1}$$

$$\Delta pH_{-43} \; = \; pH_{-4} \; - \; pH_{-3},$$

wird wie bei dem Ausführungsbeispiel nach Fig.4 die Atmung

$$\frac{\Delta pH_{-43} \; - \; \Delta pH_{-21}}{\Delta T}$$

der Biokompartimente 1 bestimmt. Zusätzlich wird aus den pH-Differenzwerten

$$\Delta pH_{+21} = pH_{+2} - pH_{+1}$$

$$\Delta pH_{+43} = pH_{+4} - pH_{+3},$$

die Konzentrationsänderung des Stickstoffmonoxids

$$\frac{\Delta pH_{+43} - \Delta pH_{+21}}{\Delta T}$$

in dem Kulturmedium bestimmt.

Außerdem werden Meßwerte

$$\Delta pH_{-31} = pH_{-3} - pH_{-1}$$

$$\Delta pH_{+31} = pH_{+3} - pH_{+1}$$

für die Ansäuerung gebildet.

In entsprechender Weise kann bei Biokompartimenten 1, die beim Stoffwechsel Wasserstoffperoxid ($H_2O_2$) aufnehmen und/oder abgeben, die durch die Biokompartimente 1 verursachte Konzentrationsänderung des Wasserstoffperoxids gemessen werden. Dabei wird während des Anlegens des positiven elektrischen Potentials im Bereich der Arbeitselektrode 19 das in dem Kulturmedium befindliche Wasserstoffperoxid entsprechend der Reaktionsgleichung

$$H_2O_2 - 2e^- \rightarrow O_2 + 2H^+$$

reduziert.

Erwähnt werden soll noch, daß die Zeitdauer, während der die elektrische Spannung an die Elektroden 19, 20 angelegt ist, so bemessen werden kann, daß die benachbart zur Arbeitselektrode 19 angeordneten Biokompartimente 1 durch den aufgrund der elektrischen Spannung im Bereich der Arbeitselektrode 19 lokal veränderten pH-Wert des Kulturmediums beinflußt werden. Dadurch ist es beispielsweise möglich, den Einfluß des pH-Wertes auf die Wirksamkeit eines in dem Kulturmedium befindlichen Zellwirkstoff zu untersuchen.

Bei dem Verfahren zur Untersuchung von biologischen Biokompartimenten 1 wird also eine die Biokompartimente 1 enthaltende Meßkammer 2 kontinuierlich oder portionsweise von Kulturmedium durchflossen. In einem benachbart zu den Biokompartimenten 1 befindlichen Kulturmediumbereich wird eine elektrische Spannung derart angelegt, daß aus einem in dem Kulturmedium befindlichen, von den Biokompartimenten abgebbaren und/oder aufnehmbaren Stoffes $OH^-$ und/oder $H^+$-Ionen gebildet werden. Während des Anlegens der Spannung wird ein erster Meßwert $pH_{-1}$, $pH_{-3}$, $pH_{+1}$, $pH_{+3}$ für den pH-Wert des Kulturmediums gemessen. Dann wird die Spannung abgeschaltet oder derart geändert, daß die Bildung von $OH^-$ und $H^+$-Ionen aus dem Stoff aufhört. Vor oder nach der Messung des ersten Meßwertes $pH_{-1}$, $pH_{-3}$, $pH_{+1}$, $pH_{+3}$ wird bei abgeschalteter elektrischer Spannung oder bei einer Spannung, bei der die Bildung von Hydroxid- und Wasserstoff-Ionen aus dem Stoff unterbleibt, ein zweiter Meßwert $pH_{-2}$, $pH_{-4}$, $pH_{+2}$, $pH_{+4}$ für den pH-Wert des Kulturmediums gemessen. Nachdem die gesamte Messung wenigstens einmal wiederholt wurde, werden aus den ersten und zweiten Meßwerten die Konzentrationsänderung des Stoffs in dem Kulturmedium und die Ansäuerung oder Basifizierung des Kulturmediums bestimmt.

**Patentansprüche**

**1.** Verfahren zur Untersuchung von membranumschlossenen Biokompartimenten (1), wobei die Biokompartimente (1) in einer Messkammer (3) bereitgestellt werden, die von einem mit den Biokompartimenten (1) in Kontakt stehenden, gegebenenfalls einen die Biokompartimente (1) beeinfussenden Wirkstoff enthaltenden Kulturmedium kontinuierlich oder portionsweise durchflossen wird, und wobei der pH-Wert des in der Messkammer (3) befindlichen Kulturmediums mittels eines ISFET gemessen wird, **dadurch gekennzeichnet,**

a) **dass** zum indirekten Messen der Konzentration eines von den Biokompartimenten (1) beim Stoffwechsel abgebbaren und/oder aufnehmbaren Stoffes in einem im Wirkungsbereich der Biokompartimente (1) befindlichen Teilbereich des Kulturmediums eine Arbeitselektrode (19) und eine dazu beabstandete Referenzelektrode (20) angeordnet werden, an die eine elektrische Spannung anlegbar ist,

b) **dass** bei abgeschalteter elektrischer Spannung oder bei einer Spannung, bei der die Bildung von Hydroxid- und Wasserstoff-Ionen aus dem in dem Kulturmedium befindlichen Stoff unterbleibt, zumindest ein erster Messwert ($pH_{-1}$, $pH_{-3}$ bzw. $pH_{+1}$, $pH_{+3}$) für den pH-Wert des Kulturmediums gemessen wird,

c) **dass** nach der Messung des ersten Messwertes ($pH_{-1}$, $pH_{-3}$ bzw. $pH_{+1}$, $pH_{+3}$) eine elektrische Spannung derart an das Kulturmedium angelegt wird, dass aus dem in dem Kulturmedium befindlichen Stoff Hydroxid-Ionen oder Wasserstoff-Ionengebildet werden,

d) **dass** in dem Teilbereich des Kulturmediums befindlichen Messstelle während des Anliegens dieser elektrischen Spannung wenigstens ein zweiter Messwert ($pH_{-2}$, $pH_{-4}$ bzw. $pH_{+2}$, $pH_{+4}$) für den pH-Wert des Kulturmediums gemessen wird,

e) **dass** aus dem ersten Messwert ($pH_{-1}$, $pH_{-3}$ bzw. $pH_{+1}$, $pH_{+3}$) und dem zweiten Messwert ($pH_{-2}$, $pH_{-4}$ bzw. $pH_{+2}$, $pH_{+4}$) die Differenz ($\Delta pH_{21}$, $\Delta pH_{43}$ bzw. $\Delta pH_{+21}$, $\Delta pH_{+43}$) gebildet wird,

f) **dass** die Schritte a) bis e) wenigstens einmal wiederholt werden,

g) **dass** aus dem Unterschied zwischen wenigstens zwei dieser Messwert-Differenzen ($\Delta pH_{21}$, $\Delta pH_{43}$ bzw. $\Delta pH_{+21}$, $\Delta pH_{+43}$) die Konzentrationsänderung des Stoffs in dem Kulturmedium und aus dem Unterschied zwischen wenigstens zwei der ersten pH-Messwerte ($pH_{-1}$, $pH_{-3}$ bzw. $pH_{+1}$, $pH_{+3}$) und/oder zweiten pH-Messwerte ($pH_{-2}$, $pH_{-4}$ bzw. $pH_{+2}$, $pH_{+4}$) die Ansäuerung oder Basifizierung des Kulturmediums ermittelt werden,

h) und **dass** aus den so gewonnenen Messwerten für die Konzentrationsänderung und die Ansäuerung oder Basifizierung die Stoffwechselaktivität der Biokompartimente (1) bestimmt wird.

2. Verfahren nach Anspruch 1, **dadurch gekennzeichnet, dass** die Abfolge bestehend aus den Schritten a) bis g) wenigstens zweimal durchlaufen wird und dass bei diesen Durchläufen die Polarität der zwischen der Arbeitselektrode (19) und der Referenzelektrode (20) angelegten elektrischen Spannung unterschiedlich gewählt wird.

3. Verfahren nach Anspruch 1 oder 2, **dadurch gekennzeichnet, dass** die Zeitpunkte für die Messung der pH-Messwerte ($pH_{-1}$, $pH_{-2}$, $pH_{-3}$, $pH_{-4}$ bzw. $pH_{+1}$, $pH_{+2}$, $pH_{+3}$, $pH_{+4}$) so auf die Zeitpunkte, an denen das elektrische Potential verändert oder abgeschaltet wird, abgestimmt werden, dass sich die Wasserstoff- und Hydroxid-Ionenkonzentration in dem Kulturmedium zu den Messzeitpunkten im wesentlichen im Gleichgewicht befindet.

4. Verfahren nach einem der Ansprüche 1 bis 3, **dadurch gekennzeichnet, dass** jeweils der Zeitabstand zwischen dem Zeitpunkt des Anlegens, Abschaltens oder Veränderns der elektrischen Spannung und dem darauf folgenden Zeitpunkt, in dem ein pH-Messwert ($pH_{-1}$, $pH_{-2}$, $pH_{-3}$, $pH_{-4}$, bzw. $pH_{+1}$, $pH_{+2}$, $pH_{+3}$, $pH_{+4}$) gemessen wird, bei allen pH-Messwerten ($pH_{-1}$, $pH_{-2}$, $pH_{-3}$, $pH_{-4}$, bzw. $pH_{+1}$, $pH_{+2}$, $pH_{+3}$, $pH_{+4}$) etwa gleich ist.

5. Verfahren nach einem der Ansprüche 1 bis 4, **dadurch gekennzeichnet, dass** die elektrische Spannung abgeschaltet oder gemäß Schritt b) angelegt wird, bevor sich ein Gleichgewichtszustand der Wasserstoff- und Hydroxid-Ionenkonzentration in dem Kulturmedium ausgebildet hat.

6. Verfahren nach einem der Ansprüche 1 bis 5, **dadurch gekennzeichnet, dass** der zwischen der Arbeitselektrode (19) und der Referenzelektrode (20) fließende elektrische Elektrodenstrom gemessen wird.

7. Verfahren nach einem der Ansprüche 1 bis 6, **dadurch gekennzeichnet, dass** die Biokompartimente (1) derart an der Arbeitselektrode (19) angelagert werden, dass sie diese zumindest bereichsweise überdecken, und dass aus der zwischen Arbeitselektrode (19) und Bezugselektrode (20) anliegenden elektrischen Spannung und dem Elektrodenstrom die Morphologie der Biokompartimente (1) ermittelt wird.

8. Verfahren nach einem der Ansprüche 1 bis 7, **dadurch gekennzeichnet, dass** der Durchfluss des Kulturmediums durch die Messkammer (3) während und zwischen der Erfassung des ersten und letzten pH-Messwerts einer zur Bestimmung der Konzentrationsänderung des Stoffs und der Ansäuerung oder Basifizierung des Kulturmediums vorgesehenen pH-Wert-Messreihe angehalten oder zumindest reduziert wird.

9. Verfahren nach einem der Ansprüche 1 bis 8, **dadurch gekennzeichnet, dass** zwischen der Erfassung des ersten und letzten pH-Messwertes der pH-Wert-Messreihe die Pufferkapazität des Kulturmediums konstant gehalten wird.

**Claims**

1. Method for investigating membrane-enclosed biocompartments (1), the biocompartments (1) being provided in a measuring chamber (3) through which flows, continuously or in portions, a culture medium which is in contact with the biocompartments (1) and, if appropriate, contains an active substance that influences the biocompartments (1), and the pH value of the culture medium situated in the measuring chamber (3) being measured by means of an ISFET, **characterized**

   a) **in that**, for indirectly measuring the concentration of a substance that can be released and/or taken up by the biocompartments (1) in the course of metabolism, in a partial region of the culture medium that is situated in the active region of the biocompartments (1), there are arranged a working electrode (19) and a reference electrode (20) spaced apart therefrom, to which an electrical voltage can be applied,
   b) **in that**, with the electrical voltage switched off or at a voltage at which the formation of hydroxide and hydrogen ions from the substance situated in the culture medium does not occur, at least one first measured value ($pH_{-1}$, $pH_{-3}$ or $pH_{+1}$, $pH_{+3}$) is measured for the pH value of the culture medium,
   c) **in that**, after the measurement of the first measured value ($pH_{-1}$, $pH_{-3}$ or $pH_{+1}$, $pH_{+3}$), an electrical voltage is applied to the culture medium in such a way that hydroxide ions or hydrogen ions are formed from the substance situated in the culture medium,
   d) **in that**, in the measuring location situated in the partial region of the culture medium, while said electrical voltage is present, at least one second measured value ($pH_{-2}$, $pH_{-4}$ or $pH_{+2}$, $pH_{+4}$) is measured for the pH value of the culture medium,
   e) **in that** the difference ($\Delta pH_{-21}$, $\Delta pH_{-43}$ or $\Delta pH_{+21}$, $\Delta pH_{+43}$) is formed from the first measured value ($pH_{-1}$, $pH_{-3}$ or $pH_{+1}$, $pH_{+3}$) and the second measured value ($pH_{-2}$, $pH_{-4}$ or $pH_{+2}$, $pH_{+4}$),
   f) **in that** steps a) to e) are repeated at least once,
   g) **in that** the change in the concentration of the substance in the culture medium is determined from the difference between at least two of these measured value differences ($\Delta pH_{-21}$, $\Delta pH_{-43}$ or $\Delta pH_{+21}$, $\Delta pH_{+43}$), and the acidification or basification of the culture medium is determined from the difference between at least two of the first pH measured values ($pH_{-1}$, $pH_{-3}$ or $pH_{+1}$, $pH_{+3}$), and/or second pH measured values ($pH_{-2}$, $pH_{-4}$ or $pH_{+2}$, $pH_{+4}$),
   h) and **in that** the metabolic activity of the biocompartments (1) is determined from the measured values thus obtained for the change in the concentration and the acidification or basification.

2. Method according to Claim 1, **characterized in that** the sequence comprising steps a) to g) is run through at least twice, and **in that** the polarity of the electrical voltage applied between the working electrode (19) and the reference electrode (20) is chosen to be different during these passes.

3. Method according to Claim 1 or 2, **characterized in that** the instants for the measurement of the pH measured values ($pH_{-1}$, $pH_{-2}$, $pH_{-3}$, $pH_{-4}$ or $pH_{+1}$, $pH_{+2}$ $pH_{+3}$, $pH_{+4}$) are coordinated with the instants at which the electrical potential is altered or switched off such that the hydrogen and hydroxide ion concentration in the culture medium is essentially at equilibrium at the measurement instants.

4. Method according to any of Claims 1 to 3, **characterized in that** the time interval between the instant of applying, switching off or altering the electrical voltage and the succeeding instant at which a pH measured value ($pH_{-1}$, $pH_{-2}$, $pH_{-3}$, $pH_{-4}$ or $pH_{+1}$, $pH_{+2}$, $pH_{+3}$, $pH_{+4}$) is measured is in each case approximately identical for all the pH measured values ($pH_{-1}$, $pH_{-2}$, $pH_{-3}$, $pH_{-4}$ or $pH_{+1}$, $pH_{+2}$ $pH_{+3}$, $pH_{+4}$).

5. Method according to any of Claims 1 to 4, **characterized in that** the electrical voltage is switched off or applied according to step b) before an equilibrium state of the hydrogen and hydroxide ion concentration in the culture medium has formed.

6. Method according to any of Claims 1 to 5, **characterized in that** the electrical electrode current flowing between the working electrode (19) and the reference electrode (20) is measured.

7. Method according to any of Claims 1 to 6,
**characterized in that** the biocompartments (1) are attached to the working electrode (19) in such a way that they cover the latter at least in regions, and **in that** the morphology of the biocompartments (1) is determined from the electrode current and the electrical voltage present between working electrode (19) and reference electrode (20).

8. Method according to any of Claims 1 to 7,
**characterized in that** the through-flow of the culture medium through the measuring chamber (3) is halted or at least reduced during and between the detection of the first and last pH measured values of a pH value measurement series provided for determining the change in the concentration of the substance and the acidification or basification of the culture medium.

9. Method according to any of Claims 1 to 8,
**characterized in that** the buffer capacity of the culture medium is kept constant between the detection of the first and last pH measured values of the pH value measurement series.

## Revendications

1. Procédé d'examen de biocompartiments (1) entourés d'une membrane, ces biocompartiments (1) étant placés dans une cellule de mesure (3) où s'écoule en continu ou par portions un milieu de culture en contact avec les biocompartiments (1) et contenant le cas échéant une substance active ayant une influence sur les biocompartiments (1) et la valeur pH du milieu de culture se trouvant dans la cellule de mesure (3) étant mesurée au moyen d'un ISFET, **caractérisé en ce que**

   a) pour la mesure indirecte de la concentration d'une substance pouvant être émise et/ou absorbée par les biocompartiments (1) lors du métabolisme, dans une sous-fraction se trouvant dans le champ d'action des biocompartiments (1) du milieu de culture, une électrode opérationnelle (19) et une électrode de référence (20) espacée par rapport à celle-ci sont disposées, une tension électrique pouvant leur être appliquée,
   b) en cas de tension électrique désactivée ou de tension avec laquelle la formation d'ions d'hydroxyde et d'hydrogène à partir de la substance se trouvant dans le milieu de culture n'a pas lieu, au moins une première valeur de mesure ($pH_{-1}$, $pH_{-3}$ ou $pH_{+1}$, $pH_{+3}$) est mesurée pour la valeur pH du milieu de culture,
   c) après la mesure de la première valeur de mesure ($pH_{-1}$, $pH_{-3}$ ou $pH_{+1}$, $pH_{+3}$), une tension électrique est appliquée au milieu de culture de manière à ce que, à partir de la substance se trouvant dans le milieu de culture, des ions d'hydroxyde ou des ions d'hydrogène se forment,
   d) au point de mesure se trouvant dans la sous-fraction du milieu de culture pendant l'application de cette tension électrique, au moins une deuxième valeur de mesure ($pH_{-2}$, $pH_{-4}$ ou $pH_{+2}$, $pH_{+4}$) est mesurée pour la valeur pH du milieu de culture,
   e) à partir de la première valeur de mesure ($pH_{-1}$, $pH_{-3}$ ou $pH_{+1}$, $pH_{+3}$) et de la deuxième valeur de mesure ($pH_{-2}$, $pH_{-4}$ ou $pH_{+2}$, $pH_{+4}$), on calcule la différence ($\Delta pH_{-21}$, $\Delta pH_{-43}$ ou $\Delta pH_{+21}$, $\Delta pH_{+43}$),
   f) les étapes a) à e) sont répétées au moins une fois,
   g) à partir de la différence entre au moins deux de ces différences entre les valeurs de mesure ($\Delta pH_{-21}$, $\Delta pH_{-43}$ ou $\Delta pH_{+21}$, $\Delta pH_{+43}$), on détermine le changement de concentration de la substance dans le milieu de culture et à partir de la différence entre au moins deux des premières valeurs de mesure de pH ($pH_{-1}$, $pH_{-3}$ ou $pH_{+1}$, $pH_{+3}$) et/ou des deuxièmes valeurs de mesure de pH ($pH_{-2}$, $pH_{-4}$ ou $pH_{+2}$, $pH_{+4}$), on détermine l'acidification ou la basification du milieu de culture,
   h) et à partir des valeurs de mesure ainsi obtenues pour le changement de concentration et l'acidification ou la basification, on détermine l'activité métabolique des biocompartiments (1).

2. Procédé selon la revendication 1, **caractérisé en ce que** la série composée des étapes a) à g) est suivie au moins deux fois et que, lors de ces passages, la polarité de la tension électrique appliquée entre l'électrode opérationnelle (19) et l'électrode de référence (20) est choisie différemment.

3. Procédé selon la revendication 1 ou 2, **caractérisé en ce que** les moments de mesure des valeurs de mesure de pH ($pH_{-1}$, $pH_{-2}$, $pH_{-3}$, $pH_{-4}$, ou $pH_{+1}$, $pH_{+2}$, $pH_{+3}$, $PH_{+4}$) sont définis en fonction des moments où le potentiel électrique est modifié ou désactivé de manière à ce que la concentration des ions d'hydroxyde et d'hydrogène dans le milieu de culture se trouve sensiblement en équilibre aux moments de la mesure.

4. Procédé selon l'une quelconque des revendications 1 à 3, **caractérisé en ce que** l'intervalle de temps entre le moment de l'application, de la désactivation ou du changement de la tension électrique et le moment suivant pendant lequel une valeur de mesure de pH ($pH_{-1}$, $pH_{-2}$, $pH_{-3}$, $pH_{-4}$, ou $pH_{+1}$, $pH_{+2}$, $pH_{+3}$, $pH_{+4}$) est mesurée est respectivement approximativement égal pour toutes les valeurs de mesure de pH ($pH_{-1}$, $pH_{-2}$, $pH_{-3}$, $pH_{-4}$, ou $pH_{+1}$, $pH_{+2}$, $pH_{+3}$, $PH_{+4}$) .

5. Procédé selon l'une quelconque des revendications 1 à 4, **caractérisé en ce que** la tension électrique est désactivée ou appliquée selon l'étape b) avant qu'un état d'équilibre de la concentration des ions d'hydrogène et d'hydroxyde dans le milieu de culture ne se soit installé.

6. Procédé selon l'une quelconque des revendications 1 à 5, **caractérisé en ce que** le courant d'électrode s'écoulant entre l'électrode opérationnelle (19) et l'électrode de référence (20) est mesuré.

7. Procédé selon l'une quelconque des revendications 1 à 6, **caractérisé en ce que** les biocompartiments (1) sont déposés au niveau de l'électrode opérationnelle (19) de manière à la recouvrir au moins par endroits et que, à partir de la tension électrique appliquée entre l'électrode opérationnelle (19) et l'électrode de référence (20) et du courant d'électrode, on détermine la morphologie des biocompartiments (1).

8. Procédé selon l'une quelconque des revendications 1 à 7, **caractérisé en ce que** le débit du milieu de culture dans la chambre de mesure (3) pendant et entre l'enregistrement de la première et de la dernière valeur de mesure de pH d'une série de mesures de valeurs de pH prévue pour déterminer le changement de concentration de la substance et l'acidification ou la basification du milieu de culture est arrêté ou au moins réduit.

9. Procédé selon l'une quelconque des revendications 1 à 8, **caractérisé en ce que**, entre l'enregistrement de la première et de la dernière valeur de mesure de pH de la série de mesures de valeurs de pH, l'aptitude à faire office de tampon du milieu de culture est maintenue constante.

Fig. 1

Fig. 2

Fig. 3

Fig. 4

Fig. 5

Fig. 6

Fig. 7

Fig. 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0394406 B1

- DE 4417078 A1

**In der Beschreibung aufgeführte Nicht-Patentliteratur**

- **WOLF, B.** Biofunctional hybrid structures - cell-silicon hybrids for applications in biomedicine and bioinformatics. *Biochemistry and Biogenergetics,* 1998, vol. 46, 215-225

- **AUS SOHN, B.-H. ; KIM, C.-S.** A new pH-ISFET based dissolved oxygen sensor by employing electrolysis of oxygen. *Sensors and Actuators,* 1996, vol. B34, 435-440